# EUROPEAN PATENT APPLICATION

(11) **EP 3 501 501 A1**
(43) Date of publication of application: **26.06.2019**
(21) Application number: 18209851.7
(22) Date of filing: 17.02.2012
(51) Int. Cl.: A61K 9/16, A61K 31/403, A61K 31/404, A61K 31/44

(54) **PHARMACEUTICAL FORMULATION HAVING IMPROVED STABILITY**

(62) Divisional of application: 12711438.7
(71) Applicant: Egis Gyógyszergyár Zrt., 1106 Budapest (HU)
(72) Inventor: FEHÉR, András, 1143 Budapest (HU); ZSIGMOND, Zsolt, 2234 Maglód (HU); ÚJFALUSSY, György, 1028 Budapest (HU); TONKA-NAGY, Péter, 1171 Budapest (HU); MOROVJÁN, György, 1196 Budapest (HU)
(74) Representative: Stolmár & Partner Patentanwälte PartG mbB

(57) **Abstract**

The present invention is related to a pharmaceutical formulation containing ramipril with improved stability, which comprises ramipril and a desintegrant directly granulated with a binder.

## Description

### Technical Field of the Invention

The present invention is related to pharmaceutical formulations containing ramipril [chemical name: (2S,3aS,6aS)-1[(S)-N-[(S)-1-carboxy-3-phenylpropyl]-alanyl]-octahydrocyclopenta-[b]pyrrole-2-carboxylic acid-1-ethylester] having improved stability and method for preparation thereof.

More specifically, the invention is related to a stabilized pharmaceutical formulation containing ramipril and a superdesintegrant formulated directly together with a binder in a granulated form.

### Background Art

Ramipril is a known pharmaceutically active ingredient which is used in the therapy for the treatment of hypertension, cardiac insufficiency and nephropathy, enhancing revascularization, decreasing the risk of cardiovascular diseases and events, especially the risk of stroke or myocardial infarction and decreasing the risk of cardiovascular mortality. Ramipril exerts its effect primarily by its active metabolite, ramiprilat, which is an especially effective inhibitor of the angiotensin converting enzyme (ACE).

Structurally analogous compounds to ramipril have been disclosed for the first time in European Patent No. 50800. Chemical structure of ramipril has been disclosed for the first time in European Patent No. 79022.

Ramipril is an easily decomposing pharmaceutically active ingredient sensitive to heat and moisture. During decomposition, hydrolysis of the ester groups, yielding the impurity having identical chemical structure to the active metabolite, occurs (European Pharmacopoeia: Impurity E). During another important decomposition reaction of ramipril, ramipril diketopiperazine is formed (European Pharmacopoeia: Impurity D). For example, according to United States Patent No. 5442008, in the case when ramipril tablets of 2.5 mg strength produced by dry granulation are subjected to storage at the temperature of 40 °C for six months, the concentration of the diketopiperazine decomposition product can reach 22.8 %. In the case of ramipril capsules having the sampe strength and stored under identical conditions, the concentration of the diketopiperazine decomposition product can reach 6.4 %. According to published International Patent Application WO2005041940, besides the decomposition reaction methods mentioned above, the oxidative decomposition of ramipril in the presence of air can result in undesired discoloration.

There are several methods known in the prior art for preventing the decomposition of ramipril. Some of these methods are based on isolating agents present in the pharmaceutical formulation or in the environment thereof which can facilitate the decomposition of ramipril. Other methods are relying on the formulation which use ingredients suitable for effectively stabilizing ramipril.

According to the methods based on the first principle, ramipril particles are provided with a coating excluding moisture and air facilitating decomposition as well as excipients present. Alternatively, the pharmaceutical dosage form is provided in a way wherein ramipril and the less compatible excipient or further active ingredient is located in a spatially separated compartment of the formulation which contacts the ramipril-containing compartment on a little surface area only.

According to United States Patent No. 5442008, ramipril particles are provided with a polymer coating and the active ingredient thus stabilized is used in subsequent formulation operations, optionally in tabletting. For the coating, hydroxypropyl-cellulose, hydroxypropyl-methyl-cellulose, a phtalate, hydroxyethyl-cellulose, ethylcellulose, cellulose-acetate-phtalate, polyvinylpyrrolidone or a metacrylate-type or a further type of pharmaceutically acceptable coating material is transferred onto the surface of ramipril particles.

Published International Patent Application No. WO2006050533 is related to ramipril particles provided with stabilizing coating wherein the aggregation before the coating operation is prevented and each particle is provided with an individual coating. As preferable coating agent, hydroxypropyl-methyl-cellulose is used. The coating process is carried out by fluidization.

According to a further method, the active ingredient is formulated in the presence of stabilizing excipients and the use of generally applied excipients incompatible with ramipril is avoided.

According to the inventors of British Patent Application No. 2394660, excipients having high specific surface area and acidic character are incompatible with ramipril, therefore their use during the formulation should be avoided. This group of excipients includes colloidal silica (Aerosil) widely used during pharmaceutical technology operations.

European Patent Application No. 1734931 is related to stable ramipril formulations containing calcium sulfate dihydrate excipient. The formulation prepared using this excipient contained 0.478-1.06 % ramipril diketopiperazine impurity after storage at 40 °C temperature and 75% relative humidity for 6 month, which is much lower than that in the case of the marketed product.

The inventors of Published International Patent Application WO 2007056907 used sodium hydrogencarbonate and calcium sulfate simultaneously for the stabilization of ramipril, whereby a decrease in the concentration of ramipril impurity was achieved. Similar method has been disclosed in Published US Patent Application No. 20030215526.

Published International Patent Application WO2005041940 discloses a formulation whererin the stabilization of an ACE-inhibitor or ramipril is attempted by using meglumine and optionally low-substituted hydroxypropyl-cellulose. The product stored at 60 °C for 15 days contained 0.75-1.59 % impurity. The disadvantage of this composition is that even short storage at elevated temperatures results in significant decomposition.

Published International Patent Application WO2005002548 is related to a stabilized pharmaceutical formulation containing an ACE-inhibitor and an effective amount of a pharmaceutical lubricant in intimate admixture besides other optional excipients. As a lubricant, any such agent known from the prior art can be used. However, the formulation according to this prior art contained similar amounts of decomposition products even after 48 hours to those of the marketed product.

In Published US Patent Application 20070232680 and Published International Patent Application WO2008000040, magnesium oxide is used for the stabilization of ramipril. Inventors of Published International Patent Application WO2008132756 used magnesium oxide and lactic acid for the same purpose. However, results of stability testing have not been disclosed in these applications.

Published International Patent Application WO2007120930 is related to stabile pharmaceutical formulations containing ramipril and a stabilizing excipient. During the stability studies of a composition consisting of ramipril (1.25 mg), gelatinized starch (23.35 mg), lactose (25.0 mg) and sodium stearyl fumarate (0.4 mg) filled into hard gelatine capsules, the inventors found that after storage at 40 °C temperature and 75% relative humidity for 3 months, the concentration of ramipril diketopiperazine impurity reached 3.5%. In the case of the original ALTACE product, under the same storage conditions and identical storage time, the concentration of the same impurity was 4.9 %.

In the case of combination formulations containing several pharmaceutically active ingredients, the incompatibility of the active ingredients has to be taken into account as well. Published International Patent Application WO2008021875 discloses a combination pharmaceutical dosage form presented as a tablet, wherein the active ingredients are located in a spatially separated layers of a multi-layer tablet, whereby the layers containing active ingredients are contacted by only minimal surface.

Published International Patent Application WO2008095263 is related to a combination pharmaceutical dosage form, wherein each of the active ingredients is present as different physical dosage forms (e.g. Powder, granules, pellets, granules, minitablets, tablets) in order to minimize the interaction between the active ingredients. Thus, for example, a tablet dosage form has been produced, wherein the fluoxetine hydrochloride active ingredient is present as an ingredient of powder mixture, while olanzapine is present in a minitablet.

According to Published International Patent Application WO2008065485, a pharmaceutical formulation containing amlodipine and an ACE-inhibitor is produced by mixing the active ingredients and adjusting the pH value of the formulation to a value higher than 6.0. This effect was achieved by mixing amlodipine besylate, microcrystalline cellulose, magnesium carbonate and a colorant, transforming the mixture thus obtained into pellets by wet granulation and filling said pellets into capsules.

Published International Patent Application WO2011104588 discloses a pharmaceutical dosage form containg ramipril and amlodipine, wherein the coated ramipril particles are used.

The composition of the marketed product TRITACE consists of sodium stearyl fumarate, hypromellose, microcrystalline cellulose, cold-swellable starch and in case of some strengths, a colorant besides the ramipril active ingredient. According to the instructions for using this product, is should be stored at a temperature not exceeding 25 °C. Thus it can be expected that the composition rapidly decomposes at higher temperature/relative humidity.

### Summary of the Invention

Health authorities responsible for the issue of marketing authorizations for pharmaceutical products set forth strict requirements regarding the active ingredient content, stability and purity of pharmaceutical products. In order to satisfy these requirements, there is a need for pharmaceutical formulations containing ramipril, which can be stored for prolonged periods without the deterioration of their quality, decrease of the active ingredient concentration below the specified limits as well as increase of the concentration of decomposition product above the limits.

Although several solution for stabilizing ramipril are known from the state of the art, in most cases, no data are provided with regard to the effects of the methods and the stability date, shelf life and impurity profile of the composition.

Accordingly, the objective of our research-development work was developing a ramipril-containing formulation which can be used as a monocomponent preparation as well as a ramipril-containing component of a combination preparation, which is compatible with the most often used pharmaceutical excipients and pharmaceutically active ingredients most often used in combination with ramipril, which can be manufactured in uniform quality at a low cost and which can be stored for a longer period of time without deterioration of quality and significant increase of the concentration of decomposition products. A further objective of the development was formulating a ramipril-comtaining composition which can be used in an amlodipine-ramipril combination product.

The above-mentioned objective is solved according to the present invention.

When mixing ramipril with excipients usually applied in the pharmaceutical technology as superdesintegrants and granulating the mixture thus obtained with a suitable binder, we have surprisingly found that a ramipril-containing pharmaceutical formulation can be obtained in form of granules or pellets which exhibits improved stability. In the formulation thus obtained, the rate of decomposition of ramipril, especially the rate of the formation of the ramiprilat decomposition product proceeds significantly slower than in other pharmaceutical compositions known from the prior art and the sum of impurities is lower as well. This recognition is very surprising since the state of the art is silent about technical solutions wherein the stabilization of an ACE-inhibitor, especially ramipril, is achieved by az excipient characterized as superdesintegrant in the state of the art.

### Detailed Description of the Invention

The new formulation according to the present invention consisting of ramipril active ingredient a superdesintegrant directly granulated with a binder is suitable for tabletting or as a capsule fill or alternatively, as a component of tabletting mixture of capsule fill, e.g. in admixture with a further vehicle or with a homogenate containing further active ingredient(s). We have found that the homogenate produced from the granules according to the present invention exhibits especially good content uniformity and thus allows for the preparation of pharmaceutical products of uniform quality.

The formulation according to the present invention can be preferably produced in form of pellets or granules, which are essentially spherical particles having approx. 0.2-2 mm particle size, which can be provided with a layer or multiple layers controlling the release of the pharmaceutically active ingredient and which can be filled into hard gelatine capsules or can be tabletted. Such preparations after administering and dispersing in the stomach into individual pellets or granules, mix well with the content of the stomach and exit from the stomach in uniform manner.

In the manufacture of the pharmaceutical formulation according to the present invention, superdesintegrants known in the state of the art can be used. Superdesintegrants are pharmaceutical excipients which, upon contact with humidity, increase their volume in a large degree ("swelling"), and such volume change result in the desintegration of the dosage form. Such superdesintegrants are, for example, certain modified starch derivatives, such as sodium starch glycolate; certain cellulose derivatives, such as low-substituted hydroxypropyl-cellulose, croscarmellose sodium; and crospovidone. Different grades of crospovidones can be especially advantageously used according to the invention. Neither the United States Pharmacopoeia, nor any other document representing the state of the art mentions the stabilizing effect of superdesintegrants or that of the preferable representatives thereof mentioned above.

According to a further aspect of the present invention, there is provided a method for the preparation of the ramipril-containing pharmaceutical formulation having improved stability, which comprises granulating ramipril and a superdesintegrant with an aqueous solution of a suitable binder, preferably a water-soluble polymer and transforming the mixture into pellets or granules. Such granulating operations can be carried out using the methods of pharmaceutical technology known from the prior art (pl. Isaac Ghebre-Sellassie: Pharmaceutical Pelletization Technology, Marcel Dekker, Inc., New York, Basel, 1989).

As a binder, any compatible binder known from the prior art, preferably a water-soluble polymer, for example, a modified cellulose derivative, the most advantageously, hydroxypropyl-methyl-cellulose can be used.

There are several methods known from the prior art suitable for producing pellets or granules, which are derived from the following three basic methods:
1. layering method,
2. extrusion-spheronization,
3. building-up granulation.

According to the layering method, the mixture consisting of the particles of the active ingredients and excipients having smaller particle size are layered upon a larger, essentially isodiametric core material, which can be either an inert core or a particle of the active ingredient. This layering can be carried out in a dragee-manufacturing vessel, in a centrifugal granulating apparatus or in a fluidization spraying equipment. In some cases, the active ingredient can be sprayed as a solution onto the surface of the inert particles.

In the extrusion-spheronization process, the active ingredient and the excipients are mixed and kneaded with a liquid and the wet mass is extruded using a tool having openings approx. 1 mm diameter, and the thus obtained extruded mass is transformed into approximately spherical shape using a rotating-plate spheronizing apparatus.

In the building-up method, the mixture of the active ingredient and the excipients are stirred in a stirred apparatus, centrifugal granulating equipment or in a fluid-bed granulating apparatus, until the particles of the powder mixture adhere to each other and transformed into spherical shape due to the shearing and abrasion forces.

The pharmaceutical formulation according to the present invention can be advantageously produced by the building-up method.

Suitable particle size of the granules or pellets filled into capsules is between 0.5 to 1.0 mm, while the preferable particle size of pellets to be compressed into tablets is between 0.3 to 0.6 mm.

During the manufacture of the stability-improved pharmaceutical formulation according to the present invention, it was especially surprising that granulation of the superdesintegrant with water was feasible despite of the large volume increase and the fact that despite of the presence of humidity during the wet-granulation process and in the swollen and moist superdesintegrant, no decomposition of the moisture- and heat-sensitive ramipril occurs either during the granulation, or during the drying of granules. During the wet granulation according to the present invention, the density of granules decreases due to the swelling of the superdesintegrant. However, during the drying step, swelling gradually decreases and the density of the granules returns to the usual operational range.

Since ramipril is a heat-sensitive active ingredient, the granulating process is carried out at a temperature below 65 °C, preferably below 50 °C, the most preferably below 45 °C.

There is no limitation regarding the ramipril concentration in the pharmaceutical formulation of improved stability according to the present invention. The ramipril concentration can be chosen, for example, between 1 and 80 weight%, preferably between 5 to 50 weight%, the most preferably, between 10 to 40 weight% according to the requirements of the finished dosage form to be produced.

If desired, the ramipril-containing, stability-improved pharmaceutical formulation can be converted into a different pharmaceutical composition after granulation by using further pharmaceutically acceptable excipients, such as fillers, binders, lubricants, glidants, desintegrants and solubility improving agents, buffers, surfactants. Thus, the final dosage form can contain, for example, fillers like calcium sulfate, calcium hydrogenphosphate, lactose, mannitol, saccharose, starch, microcrystalline cellulose; binders, such as gelatine, hydroxypropyl-cellulose, hydroxypropyl-methyl-cellulose, methylcellulose, polyvinylpyrrolidone, saccharose, starch; lubricants, such as calcium stearate, hydrogenated vegetable oil, magnesium stearate, mineral oil, sodium stearyl-fumarate.

The final dosage form can furthermore contain glidants, such as caoline, talc or silicon dioxide, disintegrants of dissolution-facilitating agents, such as alginates, sodium carboxymethyl-cellulose, crospovidone, starch, pregelatinized starch, sodium carboxymethyl starch; buffers (e.g. citrate, phosphate, carbonate or hydrogencarbonate salts), surfactants (e.g. polysorbate, sodium laurylsulfate). Examples of suitable glidants are colloidal silicone dioxide, magnesium stearate, talc or starch.
The ramipril-containing pharmaceutical formulation having improved stability according to the present invention can also transformed into a controlled release formulation. To achieve this goal, any method known from the prior art suitable for this purpose, such as coating can be applied. In the coating operation, as a film-forming agent, a water-soluble (e.g. hydroxypropyl-methyl-cellulose, polyvinyl-alcohol), a water-insoluble (e.g. ethylcellulose, ethylacrylate-methylmetacrylate copolymer, polyvinylacetate) film-forming polymer of a film-forming agent having pH-dependent solubility (e.g. cellulose-acetate-phtalate, hydroxypropyl-methyl-cellulose-acetate-succinate, ethylacrylate-metacrylic acid copolymer) can be used. Coating can be performed by using an aqueous solution or a solution made in an organic solvent of the film-forming agents or by using an aqueous dispersion thereof. Depending on the solubility characteristics of the coating, an immediate-release (water-soluble coating), extended-release (water-insoluble coating) or controlled-release (pH-dependent solubility) preparations can be produced. The quality and quantity of the coating excipient should be determined on the basis of required dissolution profile, i.e. the time-dependency of the degree of dissolution.

Pharmaceutical formulations containing a pelletized or granulated active ingredient are usually presented as dosage units. Concentration of the active ingredient during the manufacture of pellets or granules is determined by the single dose of the active ingredient present in a single dosage unit in a way that the weight of the granules or pellets containing a single dose of the active ingredient should be between 50 and 1500 mg, preferably between 100 and 700 mg.

The pharmaceutical formulation according to the present invention containing ramipril in stabilized form itself is suitable for the manufacture of finished dosage forms. However, said formulation is suitable also as a ramipril-containing component of combination pharmaceutical products. In this case, the pharmaceutical formulation according to the present invention is transformed into the finished combination dosage form using further active ingredients and optionally using further excipients.

When a second or further pharmaceutically active ingredient is to be formulated, the further active ingredients can be advantageously applied in a premixed form wherein the active ingredient is homogenized with a vehicle, e.g. microcrystalline cellulose and optionally with further pharmaceutical excipients. There are no limitations with regard to the quality if microcrystalline cellulose is used as a vehicle, different grades with different particle size and specific density can be freely used.

Using the pharmaceutical formulation according to the present invention, combination formulations of ramipril and a diuretic, e.g. hydrochlorothiazide; a calcium-channel blocker, e.g. amlodipine, felodipine or nifedipine; a beta-blocker, a cholesterol-lowering active ingredient, e.g. simvastatin or a fibrate, an alfa-adrenerg blocker, e.g. doxazosin or a platelet-aggregation inhibitor drug, can be prepared.

The stability-improved ramipril-containing pharmaceutical formulation according to the present invention can be especially advantageously used for the preparation of a combination dosage form containing ramipril and amlodipine. Amlodipine is an easily decomposing compound having poor suitability for tabletting. Amlodipine is used in pharmaceutical composition as a benzolsulfonate (besylate) salt.

It is known from Published International Patent Application WO2010038091 that in combination formulations, amlodipine may interact with a further active ingredient, which can result in undesired decomposition reaction.

Production of a stable pharmaceutical dosage form containing amlodipine is a relatively different task. In European Patent Specification No. 1458384, a tablet formulation containing amlodipine besylate has been disclosed.

Due to the hydrolysis of amlodipine besylate in the presence of moisture, it can be expected that the thus formed acidic microenvironment would result in rapid decompositon of ramipril. In the case when ramipril is used in the stability-improved formulation according to the present invention, amlodipine besylate can be added to the formulation in a simple homogenate with a vehicle, preferably microcrystalline cellulose and a lubricant, preferably magnesium stearate. We have found that in the mixture containing ramipril granules or pellets according to the present invention and amlodipine besylate obtained as described above, both ramipril and amlodipine exhibits excellent stability.

According to a further aspect of the present invention, there is provided a pharmaceutical dosage form containing ramipril and amlodipine, wherein ramipril is contained in a directly granulated form together with a superdesintegrant and a binder, preferably a water-soluble polymer and amlodipine besylate in a powder mixture along with a pharmaceutically acceptable vehicle and optionally a lubricant. As a superdesintegrant, preferably crospovidone can be used. The preferable pharmaceutically acceptable vehicle is microcrystalline cellulose.

According to a further aspect of the present invention, there is provided a method for the preparation of a stable pharmaceutical dosage form containing ramipril and amlodipine, which comprises granulating the mixture of ramipril and a superdesintegrant with a binder, preferably a water-soluble polymer, and the thus obtained particles are homogeneously admixed with a homogenate containing amlodipine besylate, pharmaceutically acceptable vehicle and optionally lubricant.

In the following further preferred embodiments of the invention are disclosed:
1. Ramipril-containing pharmaceutical formulation having improved stability, which comprises ramipril and a superdesintegrant directly granulated with a binder.
2. Pharmaceutical formulation according to embodiment 1, wherein the superdesintegrant is selected from a starch derivative, cellulose derivative or crospovidone.
3. Pharmaceutical formulation according to embodiment 1, wherein the superdesintegrant is selected from sodium starch glycolate, low-substituted hydroxypropyl-cellulose, croscarmellose sodium or crospovidone.
4. Pharmaceutical formulation according to embodiment 1, wherein the binder is a water-soluble polymer, preferably a modified cellulose derivative, the most advantageously hydroxypropyl-methyl-cellulo se.
5. Ramipril-containing pharmaceutical formulation having improved stability, which comprises ramipril and crospovidone directly granulated with hydroxypropyl-methyl-cellulose.
6. Pharmaceutical preparation comprising the ramipril-containing pharmaceutical formulation having improved stability according to any of embodiments 1 to 5 and a further pharmaceutical ingredient, e.g. a diuretic, calcium-channel blocker, beta-blocker, a cholesterol-lowering active ingredient, alpha-blocker or a platelet aggregation inhibitor.
7. Pharmaceutical preparation containing amlodipine and ramipril, which comprises the ramipril-containing pharmaceutical formulation having improved stability according to any of embodiments 1 to 5 and amlodipine, preferably in the form of amlodipine besylate.
8. Pharmaceutical preparation containing amlodipine and ramipril, which comprises ramipril-containing pharmaceutical formulation having improved stability consisting of ramipril and crospovidone directly granulated with hydroxypropy-methyl-cellulose admixed with amlodipine besylate, microcystalline cellulose and optionally a lubricant.

The comparative stability results of Example 4 demonstrate that in the combination dosage form according to Example 3, the concentration of the ramiprilat decomposition product as well as the sum of impurities (ramipril diacid, Impurity E; sum of the concentration of ramipril Impurity D and Impurity E, respectively) is much more favourable than that obtained for the formulation of the Comparative Example despite of the fact that the dosage form according to Example 3 contains amlodipine besylate besides ramipril and the stabilizing agent of the Comparative Example, sodium hydrogencarbonate is not present. Furthermore it has been concluded during the stability testing that the easily decomposing amlodipine besylate remains stable until the end of the six-month stability testing period.

Further aspects of the present invention are demonstrated by the following examples without limiting the invention to the examples only.

### Comparative Example

The composition of the pharmaceutical formulation disclosed in Published International Patent Application WO2007120930 has been reproduced whith the modification that the composition has been supplemented with sodium hydrogencarbonate known from the prior art as a stabilizing agent.

The composition of the pharmaceutical formulation: ramipril 3.85%, sodium hydrogencarbonate 3.85%, lactose 74.3%, croscarmellose sodium 2.00%, pregelatinized starch 15%, sodium stearyl fumarate 1%.

For the production of 20000 tablets of 2.5 mg strength, 3.1 kg of lactose monohydrate, 50 g of ramipril, 50 g of sodium hydrogencarbonate, 80 g of croscarmellose sodium and 600 g pregelatinized starch are mixed, sieved and granulated with 1600 g of 1:1 (v/v) mixture of 96% (v/v) ethanol and water. Granules are sieved and dried at a temperature between 40 and 50 °C until 1.7% moisture content. The granules are mixed with 40 g of sieved sodium stearyl fumarate and tabletted using a rotating punch tabletting machine. Formulation is carried out in an environment where the relative moisture content of the air is lower than 40%.

### Example 1. Ramipril granules

For manufacturing 10.5 kg ramipril granules, a Hüttlin Pilotmix vortex granulating apparatus is charged with 2000 g of ramipril and 8000 g of crospovidone (Poliplasdone XL-10GAF) and the mixture is homogenized at 15 rpm for 2 minutes.

The day before actually performing the manufacturing, 470 g of hypromellose (Pharmacoat 603) are dissolved in 7.7 kg of purified water and the thus obtained granulating solution is filtered.

A 3670-g portion of the granulating solution is sprayed onto the powder mixture at 30 to 50 rpm mixer and 1000 to 1400 rpm chopper setting at a spray pressure of 1.5 bar. Following the spraying, the mixture is kneaded for further 5 minutes.

The wet product is sieved and regranulated using a Glatt OR5030 oscillating regranulation apparatus using a 0.8-mm mesh sieve.

The pregranulated mixture is transferred into the container of a Hüttlin Pilotlab fluid granulating apparatus and further granulated by spraying the remaining part of the granulating solution thereonto. During the fluid granulation operation, volumetric flow rate of fluidizing air was 800 m³/h. Air temperature was 50 °C, mass flow of granulating solution during spraying was 0.2 kg/min, spray pressure was 0.7 bar. When granulation is complete, granules are dried in the fluid granulating apparatus at a temperature not exceeding 50 °C until moisture content is between 2 and 3.5 % and the product is regranulated using a Glatt oscillating regranulation apparatus with 0.8-mm mesh sieve.

### Example 2. Ramipril capsules

1047 g of granules having ramipril as active ingredient produced according to the method of Example 1 are homogenized with 1671 g of microcrystalline cellulose (Vivapur 200).

The mixture is sieved using 0.8-mm mesh sieve size and after the addition of 3000 g microcrystalline cellulose (Vivapur 200), homogenized in a drum homogenizer for 10 minutes.

82 g of glycerol-dibehenate (Compritol 888) and 200 g of microcrystalline cellulose are mixed and sieved using 0.8-mm mesh sieve size. The mixture is added to the ramipril-containing mixture and honogenized in a drum for 2 minutes. The product thus obtained contains 1 mg ramipril in each 30 mg of the mixture.

### Example 3. Homogenate containing ramipril and amlodipine

1390 g of amlodipine besylate and 1964 g of microcrystalline cellulose (Vivapur 200) are mixed and sieved using a 0.8-mm mesh sieve. The mixture thus obtained is homogezined with 5236 g of ramipril granules produced according to Example 1 and 5000 g of miccrocrystalline cellulose (Vivapur 200) in a drum homogenizer for 10 minutes.

The product of homogenization is sieved using a 0.8-mm mesh sieve on a Glatt OR5030 oscillating regranulation apparatus and after addition of 15 kg microcrystalline cellulose (Vivapur 200), repeatedly homogenized for 10 minutes.

410 g of glycerol-dibehenate (Compritol 888) and 1000g microcrystalline cellulose are mixed and sieved using a 0.8-mm mesh sieve, and the lubricant mixture thus obtained is homogenized with the amlodipine besylate- and ramipril-containing mixture and homogenized in a drum homogenizer for 2 minutes. The product thus obtained contains 1 mg ramipril and 1 mg of amlodipine in each 30 mg and can be directly used for filling capsules.

### Example 4. Stability testing

The concentration of ramipril-diketopiperazine impurity (European Pharmacopoeia Impurity D) and ramipril diacid (European Pharmacopoeia Impurity E, ramiprilat) in the compositions manufactured according to the Comparative Example and Example 3 has been determined in a 6-month stability study carried out at the conditions 25 °C /60 % relative humidity and 40 °C/ 75 % relative humidity, respectively. The concentration of the impurities have been determined using analytical methods known in the prior art.

Stability results - Ramipril (r.h.: relative humidity)

| | 6 months | | | |
|---|---|---|---|---|
| | Composition according to Example 3 | | Composition according to Comparative Example | |
| | 25°C/60% r.h. | 40°C/75% r.h. | 25°C/60% r.h. | 40°C/75% r.h. |
| Impurity D | 0.73 | 2.00 | 0.48 | 0.83 |
| Impurity E | 0.05 | 0.07 | 3.42 | 6.0 |

The concentration of the main decomposition product of amlodipine besylate, (3-ethyl-5-methyl-2-[(2-aminoethoxy)methyl]-4-(2-chlorophenyl)-6-methylpyridine-3,5-dicarboxylate (European Pharmacopeia: Impurity D) did not exceed the 0.05 % concentration at any storage condition during the 6-month stability testing period.

## Claims

1. Pharmaceutical formulation in form of granules or pellets consisting of ramipril as active ingredient, a superdisintegrant and a binder.

2. Pharmaceutical formulation according to claim 1, **characterized by** that the ramipril concentration thereof is between 1 and 80 weight%, preferably 10 and 40 weight%.

3. Pharmaceutical formulation according to any of claims 1 to 2, **characterized by** that the granules or pellets are coated.

4. Pharmaceutical formulation according to claim 1, **characterized by** that the superdisintegrant is selected from a starch derivative, a cellulose derivative or crospovidone.

5. Pharmaceutical formulation according to claim 1, **characterized by** that the superdisintegrant is selected from sodium starch glycolate, low-substituted hydroxypropyl-cellulose, croscarmellose sodium or crospovidone.

6. Pharmaceutical formulation according to claim 1, **characterized by** that the binder is a water-soluble binder, preferably hydroxypropyl-methyl-cellulose.

7. Pharmaceutical formulation according to claim 1, **characterized by** that it consists of about 200 weight parts of ramipril, 800 weight parts of crospovidone and 47 weight parts of hydroxymethyl-propyl-cellulose.

8. Pharmaceutical dosage form comprising the pharmaceutical formulation according to claim 1 and further excipients filled into capsules or in tablet form.

9. Pharmaceutical dosage form comprising the pharmaceutical formulation according to claim 1 and a second active ingredient selected from duiretics, calcium-channel blockers, beta-blockers, cholesterol-lowering active ingredients, alpha-blockers or platelet-aggregation inhibitors.

10. Pharmaceutical dosage form according to claim 9, **characterized by** that the second active ingredient is amlodipine besylate.
